# EUROPEAN PATENT SPECIFICATION

(11) **EP 2 081 029 B1**
(45) Date of publication and mention of the grant of the patent: **26.12.2012**
(21) Application number: 09000609.9
(22) Date of filing: 16.01.2009
(51) Int. Cl.: G01N 33/68

(54) **Bio-markers for diagnosing diabetic retinopathy**
Biomarker zur Diagnose diabetischer Retinopathie
Bio-marqueurs pour le diagnostic de rétinopathie diabétique

(30) Priority: 17.01.2008 KR 20080005162
(43) Date of publication of application: 22.07.2009
(60) Divisional application: 10075360.7
(73) Proprietor: Korea University Research and Business Foundation, Seoul 136-713 (KR)
(72) Inventor: Kim, Chan Wha, Seoul 140-744 (KR); Yoo, Hyunsyuk, Seoul 157-840 (KR); Kim, Jae-Chan, Seoul 138-768 (KR); Kim, Pan Kyeom, Seoul 136-766 (KR); Park, Hye Won, Seoul 138-796 (KR); Kim, Mi Ryung, Goyang-si Gyeonggi-do 410-742 (KR)
(74) Representative: Grünecker, Kinkeldey, Stockmair & Schwanhäusser

(56) References cited:
- WO-A-2004/039956
- WO-A-2005/029088
- WO-A-2007/089731
- HERBER SIMONE ET AL: "Two-dimensional analysis of tear protein patterns of diabetic patients" ELECTROPHORESIS, vol. 22, no. 9, May 2001 (2001-05), pages 1838-1844, XP002519326 ISSN: 0173-0835

## Description

### FIELD OF THE INVENTION

The present invention relates to an in vitro method of diagnosing diabetic retinopathy.

### BACKGROUND OF THE INVENTION

Diabetes mellitus (DM), a representative metabolic disorder caused by long-term maintenance of abnormally high blood glucose (sugar) level, is a systemic, non-contagious, chronic disease.

Diabetes mellitus develops due to an absolutely or relatively diminished production of insulin induced by autoimmune destruction of insulin-producing beta cells of the pancreas, or decreased effects of insulin in a target organ (insulin resistance), which causes consistent increase of blood glucose level and impairs body metabolism including glucose metabolism.

Diabetic retinopathy is one of the major microvascular complications of diabetes mellitus, which is caused by inharmonious supply of blood to the retina due to the circulatory disturbance resulting from the gradual deformation and occlusion of the retinal microvessles on account of the consistently high blood glucose level and metabolic abnormality caused thereby. Because the frequency of occurrence of diabetic retinopathy increases as the period suffering from diabetes mellitus is long and there are no warning signs for some time, it is necessary to periodically examine the change of the retina in a diabetic patient.

Recently, there have been increasing needs for developing diagnostic markers for the early diagnosis of diabetic retinopathy and an effective method for diagnosing diabetic retinopathy. Accordingly, many proteins have been presented as markers for diagnosing diabetic retinopathy, examples of such proteins including vascular adhesion molecules such as endothelin-1(ET-1), protein kinase C(PKC) and E-selectin; thrombin-cleaved osteopontin; phosphoinositides; thrombin-stimulated platelet aggregation; vitreous VEGF; circulating soluble ICAM-1 (sICAM-1); advanced glycation end products(AGEs); nitric oxide; cytokines such as IL-1, IL-6, IL-8 and tumor necrosis factor-α; HLA alleles such as HLA-DR1, HLA-A9 and HLA-B40; glycosylated haemoglobin(HbA1c); microalbuminurea; aldose reductase(AR); Fas; PEDF; and the like proteins. However, none of the above proteins showed specificity for diabetic retinopathy only.

Hitherto, a serum has been typically used as a source of biomarkers for the early diagnosis of diabetic retinopathy. However, there have been difficulties in finding out such biomarkers from a serum because of the glycosylation of serum proteins and presence of other abundant proteins such as albumin and immunoglobulin.

Generally, tears consist of mucins, lipids, salts, glycoproteins and other various proteins. Representative tear proteins include lysozyme, lactoferrins, secretory immunoglobulin A (sIgA), lipocalins, albumins and lipophilins. Any quantitative or qualitative changes of these proteins may be interpreted as changes in the pathological conditions of our bodies. Although there have been many attempts to analyze tear proteins in relation with a disease, most of them are limited to discover the relationship between a single protein with a specific disease.

WO 2004/039956 A relates to compositions comprising a polypeptide or an antibody specific for the polypeptide, and methods useful for the diagnoses and treatment of immune related diseases such as diabetes mellitus. A kit comprising an antibody specific for the polypeptide is also disclosed.

The present inventors have endeavored to analyze the tear proteins more accurately and efficiently, and to develop effective biomarkers for early diagnosis of diabetic retinopathy. Finally, the present inventors have succeeded in accurately and efficiently analyzing tear proteins by employing two-dimensional gel electrophoresis (2-DE), thereby finding out many biomarker proteins for the diagnosis of diabetic retinopathy. Further, the present inventors have developed a composition for treating or preventing diabetic retinopathy, which comprises the biomarker proteins, and a composition and a kit for diagnosing diabetic retinopathy, which comprises an antibody specific for any one of the biomarker proteins.

### SUMMARY OF THE INVENTION

Accordingly, it is an object of the present invention to provide a method of diagnosing diabetic retinopathy.

Described are proteins, or immunogenic fragments thereof for diagnosing diabetic retinopathy.

Further described is a composition for diagnosing diabetic retinopathy, comprising antibodies specific for the proteins or the immunogenic fragments thereof.

Further described is a kit for diagnosing diabetic retinopathy, comprising antibodies specific for the proteins or the immunogenic fragments thereof.

Also described is a composition for treating or preventing diabetic retinopathy, comprising the proteins or the immunogenic fragments thereof as an active ingredient.

### BRIEF DESCRIPTION OF THE DRAWINGS

The above and other objects and features of the present application and the present invention will become apparent from the following description of the invention, when taken in conjunction with the accompanying drawings, which respectively show:
Fig. 1A is a 2-DE gel image of the proteins expressed in the tears of healthy person;
Figs. 1B and 1C is 2-DE gel images of the proteins down-regulated or up-regulated in the tears of a non-retinopathic diabetic patient and an NPDR patient, respectively;
Fig. 2A is magnified images of the proteins shown in Figs. 1A to 1C;
Fig. 2B is relative intensities of the proteins shown in Figs. 1A to 1C;
Fig. 3A is a western blot analysis result of LCN-1, Hsp27 and B2M identified in the tears of a patient with diabetic retinopathy; and
Figs. 3B to 3D display relative intensities of LCN-1, Hsp27 and B2M, respectively, in healthy persons, non-retinopathic diabetic patients and NPDR patients.

In the above figures, Healthy means a healthy person; No DMR, a non-retinopathic diabetic patient; and NPDR, a NPDR patient;

### DETAILED DESCRIPTION OF THE INVENTION

As used herein, the expression "peptide sequence obtained by trypsin digestion" means any peptide formed when a protein is digested by trypsin. Further, the molecular weight and isoelectric point (PI) of the proteins are measured by 2-dimentional electrophoresis, which may comprise an allowable experimental error.

As used herein, the term "non-retinopathic diabetic patient" means a patient suffering from diabetes mellitus (DM), e.g., type 2 DM without retinopathic symptoms, and the term "NPDR patient" means a patient suffering from type 2 DM with the symptoms of non-proliferative diabetic retinopathy (NPDR).

As used herein, the term "immunogenic fragment" means a fragment of a protein, which has at least one epitope being recognized by an antibody specific for the protein.

The present invention provides an vitro method of diagnosing diabetic retinopathy, which comprises measuring the expression level of a DJ-1 protein having a molecular weight of 24 to 26 kDa and an isoelectric point (PI) of 5.83 to 6.83. which comprises the peptide sequence of SEQ ID NOs: 1 and 2 obtained by trypsin digestion thereof, in the tears of a subject, and comparing the measured expression level to that measured in a healthy subject not suffering from diabetes.

Specifically, the method for diagnosing diabetic retinopathy of the present invention comprises 1) subjecting the tears obtained from a subject to a reaction with an antibody specific for the biomarker protein, to obtain an antigen-antibody complex; 2) adding a secondary antibody conjugated with a marker, which will develop a color upon reaction with a substrate thereof and a solution comprising the substrate to detect the antigen-antibody complex by color reaction; and 3) comparing the detected amount of the protein in the subject with that detected in a healthy subject not suffering from diabetes.

In step 1), the reaction of the tears of the subject with the antibody may be performed in a nitrocellulose membrane, or 96-well plate made of polyvinyl resin or polystyrene resin, or on a slide glass.

The subject may be diagnosed as having diabetic retinopathy when the expression level of a DJ-1 protein in the subject's tears is higher by at least 100%, preferably at least 200%, and more preferably at least 300%, than that of non-retinopathic diabetic patients.

The following Examples are intended to further illustrate the present invention and the present application without limiting its scope.

### Example 1: Analysis of tear proteins by 2-dimensional electrophoresis

### Step 1: Sample preparation

Tear fluid samples were obtained from the meniscus of 14 healthy persons (control), 10 patients suffering from type 2 DM without retinopathy (hereinafter, referred to as "non-retinopathic diabetic patients"), and patients suffering from type 2 DM with NPDR (hereinafter, referred to as "NPDR patients") by using polyester wicks. The polyester wicks thus obtained were placed on the top of 1.5 ml of micropipet tips and centrifuged at 10,000 ppm for 5 min (Union-55R centrifuge, Hanil science, Korea). The concentration of the tear proteins was measured by the Bradford method, and then the tear proteins were stored at -70°C until further use.

### Step 2: 2-Dimensional electrophoresis (2-DE)

### (2-1) Isoelectrofocusing (IEF)

60 µg each of the tear proteins obtained in step 1 was subjected to an isoelectrofocusing. 450 µℓ of rehydration buffer solution (8M urea, 2% CHAPS, 13mM DTT, 1% IPG buffer and Bromophenol blue (BPB)) was added to the tear protein sample. The mixture was loaded in a 24 cm strip holder, and Drystrips (Amerahsm Pharmacia Biotech, Uppsala, Sweden) having a pH ranging from 4 to 7 was placed in the strip holder. The strips were rehydrated for 5 hours without a current, and then, for another 5 hours with a current of 80V Isoelectrofocusing (IEF) was carried out for a total of 100,000 Vhr using an IPGphore IEF system (Amerahsm Pharmacia Biotech, Uppsala, Sweden).

### (2-2) Sodium dodecyl sulfate polyacrylamide gel electrophoresis (SDS-PAGE)

Following IEF separation, the gel strips obtained from (2-1) were equilibrated with 10 mℓ of the first equilibration solution (6M urea, 50 mM Tris-Cl buffer (pH 8.8), 20% glycerol, 2% SDS and 1% DTT) for 15 min, and the first equilibration solution was removed. The gel strips were then equilibrated again with 10 mℓ of the second equilibration solution (6M urea, 50 mM Tris-Cl buffer (pH 8.8), 30% glycerol, 2% SDS and 1.5% IAA) for 10 min. The strips were applied onto 11-16% gradient of polyacryl amide gel (25×30 cm), and subjected to a sealing with 0.5% agarose gel. SDS-PAGE was conducted by adding a buffer solution (24 mM Tris, 192 mM glycine and 0.1% SDS) to an Ettan DALT system (Amerahsm Pharmacia Biotech, Uppsala, Sweden) at a current of 70 V for 1 hour, 140 V for 2 hours, and 320 V for 5 hours.

### (2-3) Staining

In order to visualize the gels obtained in (2-2), a silver staining was conducted. The gels obtained in (2-2) were fixed in a mixture of 50% methanol, 12% acetic acid and 0.05% formaldehyde for at least 2 hours. The fixed gels were rinsed with 50% ethanol three times for 20 min each, then sensitized with 0.2% sodium thiosulfate for 1 min and followed by washing with D.W. The gels were immersed in a mixture of 0.1% silver nitrate and 0.075% formaldehyde (37%) for 20 min, and washed with D.W. The resulting gels were developed with a mixture of 6% sodium carbonate and 0.075% formaldehyde (37%) for 7 min, and 50% methanol and 12% acetic acid were added thereto to fix the resulting gels.

### (2-4) Image Analysis

In order to analyze images of the silver-stained gels, the gels were scanned by an Image Scanner (Amersham Pharmacia Bio-tech, Uppsala, Sweden) The patterns of the spots were automatically analyzed using Image-Master 2D Platinum version 6.0 (GE Healthcare, UK).

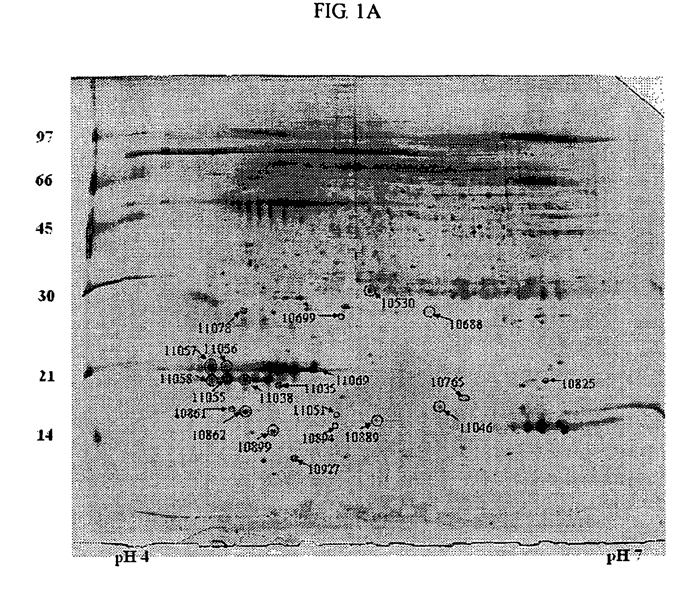

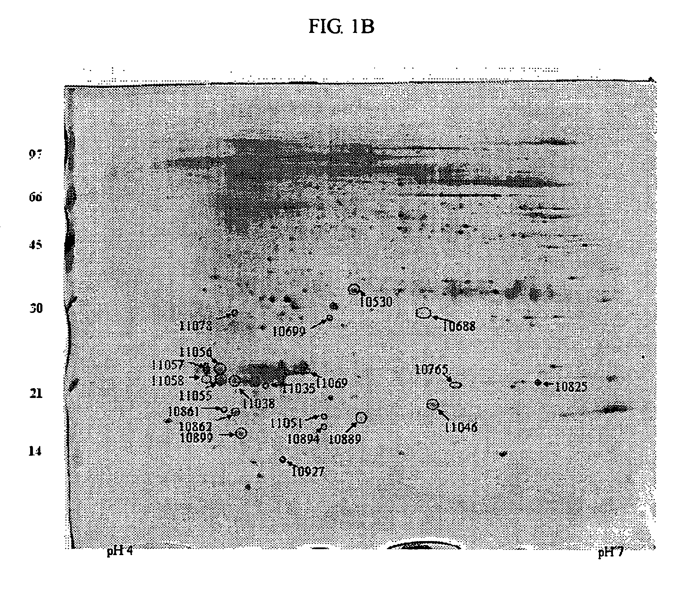

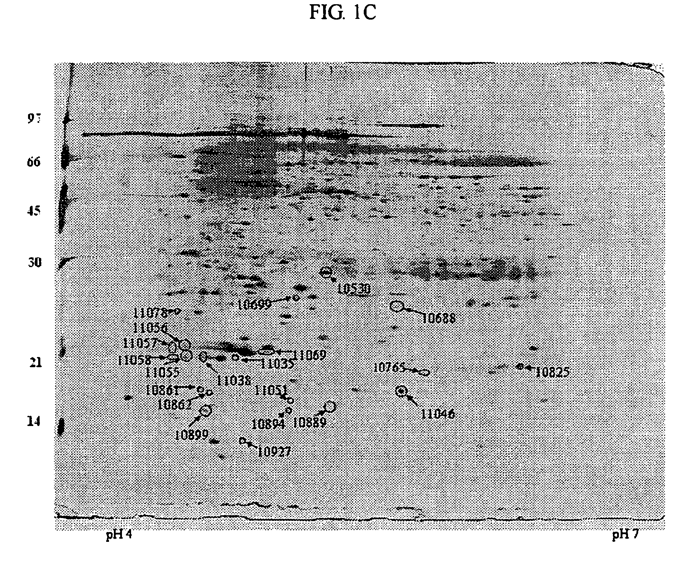

The images of SDS-PAGE electrophoresis of the proteins expressed in the tears of a healthy person (control), and a non-retinopathic diabetic patient and a NPDR patient are shown in FIGs. 1A to 1C, respectively.

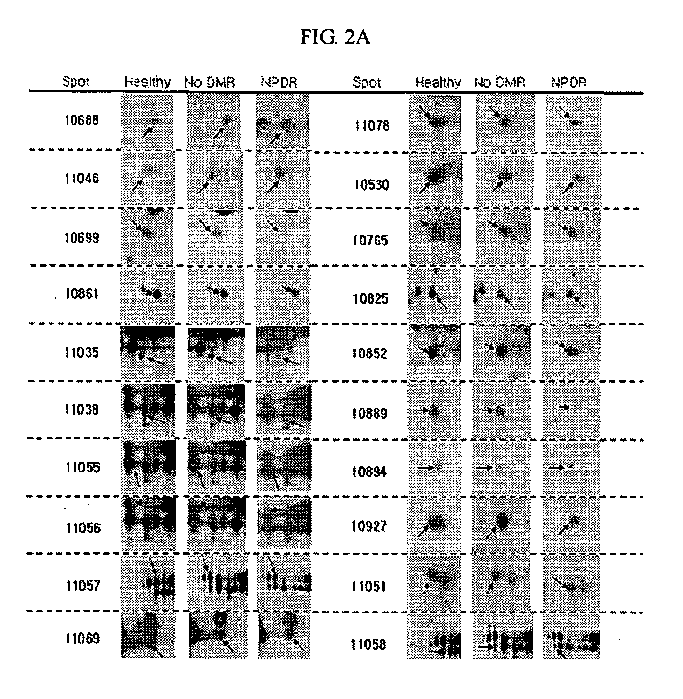

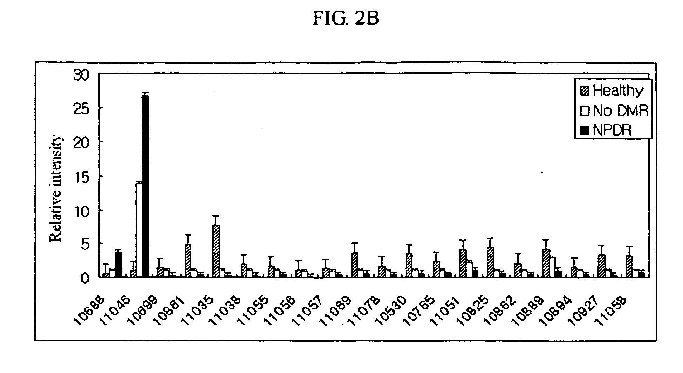

Also, the magnified images of the proteins shown in FIGs. 1A to 1C are shown in FIG. 2A, the relative intensities of the proteins are shown in FIG. 2B. Wherein Healthy is a healthy person group, No DMR is a non-retinopathic diabetic patient group; and NPDR is a NPDR patient group.

As shown in FIGs. 1A to 2B, newly expressed proteins and up-regulated or down-regulated proteins were observed in the samples of the NPDR patients as compared with those of the healthy persons or the non-retinopathic diabetic patients. Specifically, 18 proteins showing down-regulated expressions (spot Nos. of 10699, 10861, 11035, 11038, 11055, 11056, 11057, 11069, 11078, 10530, 10765, 11051, 10825, 10852, 10889, 10894, 10927 and 11058), and 2 proteins showing up-regulated expressions (spot Nos. 10688 and 11046) were found in the NPDR patients as compared with the healthy persons or non-retinopathic diabetic patients (*see,* FIGs. 1A to 2B). In the NPDR patients, the expression of 18 proteins were down-regulated by at least 50%, and that of 2 proteins were up-regulated by at least 300%, as compared with the healthy persons or the non-retinopathic diabetic patients.

The molecular weight and the isoelectric point (PI) of the spots of these proteins are shown in Table 1.

**<Table 1>**

| Spot No. | Molecular weight (kDa) | pI | classification |
|---|---|---|---|
| 10688 | 20/25 | 6.33 | Up-regulated |
| 11046 | 13/16 | 5.77 | Up-regulated |
| 10699 | 54/28 | 7 | Down-regulated |
| 10861 | 14/20 | 4.74 | Down-regulated |
| 11035 | 18/21 | 4.93 | Down-regulated |
| 11038 | 19/21 | 4.8 | Down-regulated |
| 11055 | 45/21 | 5.14 | Down-regulated |
| 11056 | 21/22 | 4.63 | Down-regulated |
| 11057 | 10/22 | 6.51 | Down-regulated |
| 11069 | 19/22 | 5.39 | Drown-regulated |
| 11078 | 48/28 | 4.84 | Drown-regulated |
| 10530 | 22/31 | 7.83 | Down-regulated |
| 10765 | 19/20 | 5.78 | Down-regulated |
| 11051 | 14/20 | 5.3 | Down-regulated |
| 10825 | 16/19 | 6.46 | Down-regulated |
| 10852 | 16/19 | 7 | Down-regulated |
| 10889 | 13/15 | 5.71 | Down-regulated |
| 10894 | 13/14 | 5.71 | Down-regulated |
| 10927 | 12/12 | 6.78 | Down-regulated |
| 11058 | 14/21 | 5.2 | Down-regulated |

### Example 2: Identification of proteins by ESI-Q-TOF MS/MS analysis

To identify the proteins obtained in Example 1, the gel spots were excised. The excised gel spots were destained with 100 µ L of destaining solution comprising 30 mM of potassium ferricyanide and 100 mM sodium thiosulfate as a ratio of 1:1 (v/v) for 5 min. After the gel spots were washed three times with 400 µℓ of water, the gel spots were incubated with 200 mM ammonium bicarbonate for 10 min, and washed again. The resulting gel spots were dehydrated with acetonitrile, until the gel was changed to be a white, and dried with a speed vacuum centrifuge. The dried gel spots were the rehydrated with 20 µℓ of 50 mM ammonium bicarbonate containing 0.2 µg of trypsin (promega) for 45 min on ice. After the removal of the solution, 30 µℓ of 50 mM ammonium bicarbonate was added thereto, and incubated overnight at 37°C for digestion. A peptide solution obtained in above was desalted using C¹⁸ nano column (homemade).

Custom-made chromatographic columns were employed for desalting and concentraion of the peptide solution, prior to mass spectrometric analysis. A column consisting of 100-300 nL of Poros reverse phase R2 material (20-30 um bead size, PerSeptive Biosystems) was packed into a constricted GELoader tip (Eppendorf, Hamburg, Germany). A 10 mL syringe was then utilized to force liquid through the column, by applying a gentle air pressure. 30 µℓ of the peptide solution from the digestion supernatant was then diluted, loaded onto the column, and washed with 20 µℓ of 5% formic acid. For MS/MS analysis, the peptides were eluted with a mixture of 50% methanol, 49% H₂O and 1% formic acid directly into a precoated borosilicate nanoelectrospray needle (Micromass, Manchester, U.K.).

The MS/MS analysis of the peptides was performed by nano-ESI on a Q-TOF mass spectrometer (Micromass, Manchester, U.K.). The source temperature was 80°C. A 1 kV potential was applied to precoated borosilicate nanoelectrospray needles (EconoTip, New Objective, USA) in the ion source, combined with a nitrogen back-pressure of 0-5 psi, to achieve a stable flow rate (10-30 nL/min). The cone voltage was 40 V. The quadrupole analyzer was used to select precursor ions for fragmentation in the hexapole collision cell. The collision gas used was argon (Ar) at a pressure of 6-7×10⁻⁵ mbar, and a collision energy was 20-30V. The product ions were analyzed with an orthogonal TOF analyzer, fitted with a reflector, a microchannel plate detector and a time-to-digital converter. The data was processed using a Mass Lynx Windows NT PC system (Micromass, Manchester, U.K.).

For identification of the protein, all MS/MS spectra recorded on the tryptic peptides derived from the spots were searched against the protein sequences from the NCBInr databases (www.ncbi.nlm.nih.gov), using the MASCOT search program (www.matrixscience.com). The results of identification of the proteins up-regulated or down-regulated, are shown in Tables 2 and 3, respectively.

**<Table 2>**

| Spot NO. | Identification of protein | MW (Da) | Peptide by trypsin digestion | | NCBI Assession No. | SEQ ID NO. |
|---|---|---|---|---|---|---|
| | | | Sequence | SEQ ID NO. | | |
| 10688 | DJ-I protein | 20050 | GAEEMETVIPVDVMR | 1 | NP_009193 | 33 |
| | | | EGPYDVVVLPGGNLGAQNLSESAAVK | 2 | | |
| 11046 | Beta-2 microglobulin | 12905 | SNFLNCYVSGFHPSDIEVDLLK | 3 | CAA23830 | 34 |
| | | | DWSFYLLYYTEFTPTEKDEYACR | 4 | | |

**<Table 3>**

| Spot NO. | Identification of protein | MW (Da) | Peptide by trypsin digestion | | NCBI Assession No. | SEQ ID NO. |
|---|---|---|---|---|---|---|
| | | | Sequence | SEQ ID NO. | | |
| 10699 | Envelope protein | 54093 | SGLNTEAFYVMTVGSK | 5 | AAB05390 | 35 |
| | | | | 6 | | |
| 10861 | Protein SAP1 | 13491 | IIPGGIYDADLNDEWVQR | 7 | AAB19889 | 36 |
| 11035 | Lipocalin 1-like 1 | 18078 | GLSTESILIPR | 8 | CAH73781 | 37 |
| 11056 | Lipocalin 1-like 1 | 18078 | GLSTESILIPR | 8 | CAH73781 | 37 |
| 11038 | Lipocalin (hCG201503) | 19488 | GLSTESILIPR | 8 | EAW88053 | 38 |
| 11055 | Cytokeratin 4 | 45593 | FASFIDKVQFLEQQNK | 9 | CAA30534 | 39 |
| | | | WNLLQQQTTTTSSK | 10 | | |
| | | | NLEPLFETYLSVLR | 11 | | |
| | | | VDSLNDEINFLK | 12 | | |
| | | | NLDLDSIIAEVR | 13 | | |
| | | | VQQLQISVDQHGDNLK | 14 | | |
| 11057 | S 100 calcium-binding protein A8 | 10885 | ELDINTDGAVNFQEFLILVIK | 15 | NP_002955 | 40 |
| 11069 | Lipocalin 1 precursor | 19409 | GLSTESILIPR | 8 | NP_002288 | 41 |
| 11078 | Keratin 4 | 48633 | QNQEYQVLLDVR | 16 | NP_002268 | 42 |
| | | | LNVEVDAAPTVDLNR | 17 | | |
| 10530 | Heat shock protein 27 | 22427 | LFDQAFGLPR | 18 | AAA62175 | 43 |
| | | | VSLDVNHFAPDELTVK | 19 | | |
| | | | LATQSNEITIPVRFESR | 20 | | |
| 10765 | Adenine phospho-ribosyl-transferase isoform | 19766 | LQAEVLECVSLVELTSLK | 21 | NP_000476 | 44 |
| 11051 | Phosphohistidine phosphatase | 14 kDa | ALIPDVDLDSD | 22 | BC024648 | 45 |
| 11058 | Phosphohistidine phosphatase | 14 kDa | ALIPDVDLDSD | 22 | BC024648 | 45 |
| 10825 | Beta globin | 16101 | LLVVYPWTQR | 23 | AAF00488 | 46 |
| | | | EFTPPVQAAYQK | 24 | | |
| | | | FFESFGDLSTPDAVMGNPK | 25 | | |
| 10852 | Lysozyme precursor | 16885 | GISLANWMCLAK | 26 | 1LHM | 47 |
| | | | STDYGIFQINSR | 27 | | |
| 10889 | S 100 calcium binding protein A9 | 13291 | QLSFEEFIMLMAR | 28 | NP_002956 | |
| | | | DIETIINTFHQYSVK | 29 | | 48 |
| | | | VIEHIMEDLDTNADKQLSFEEFIMLMAR | 30 | | |
| 10894 | Crystal structure of human calprotectin | 13086 | QLSFEEFIMLMAR | 31 | NP_002956 | 49 |
| 10927 | S100 calcium binding protein A4 | 11949 | ALDVMVSTFHK | 32 | AAB20971 | 50 |

### Example 3: Western blotting

### (3-1) SDS-PAGE electrophoresis

40-50 µg of the proteins obtained from the tears of the non-retinopathic diabetic patients and NPDR patients were diluted with SDS-PAGE loading buffer (60 mM Tris-Cl, 2% SDS, 25% Glycerol, 14.4 mM 2-mercaptoethanol and 0.1% bromophenol blue; pH 6.8), and subjected to 12% SDS-PAGE. The gels were blotted onto mini gel (6×8cm) using a running buffer at a current of 120V for molecular weight separation. The running buffer is containing 0.025M Tris-Cl, 0.192M Glycine and 1% SDS (pH 8.3).

### (3-2) Blotting

The gels obtained in (3-1) were transferred onto nitrocellulose (NC) membranes to blot using semi-dry transfer kit (Bio-rad) at a current of 50mA for 1 hour 30 min.

### (3-3) Antibody reaction

An antibody reaction was conducted by employing LCN-1 (Spot NO. 11038), Hsp27 (Spot NO. 10530) and B2M (Spot NO. 11046) obtained from the NPDR patients. The nitrocellulose membranes were incubated with blocking solution of 5% w/v nonfat dry milk for 1 hour at room temperature. Anti-LCN1-monoclonal (1:1000), Anti-Hsp27-monoclonal (1:1000) and Anti-B2M-monoclonal (1:500) antibodies were used with the blocking solution, and incubated at 4 °C for 24 hours. The monoclonal antibody prepared by a conventional hybridoma technique (Koeher and Milsteinas (1975) Nature, 256:495) using a rabbit was used as a primary antibody. The resulting solution was washed with TBS/T (0.1% Tris-buffered saline-tween20), and subsequently incubated with a solution containing 1:10,000 of horseradish peroxidase (HRP)-conjugated anti-rabbit secondary antibody (Santa cruz biotechnology Incs.) at a room temperature for I hour. The resulting solution was washed with TBS/T, and detection was performed with Enhanced chemiluminescence (ECL) system (Pierce Biotechnology Inc., USA).

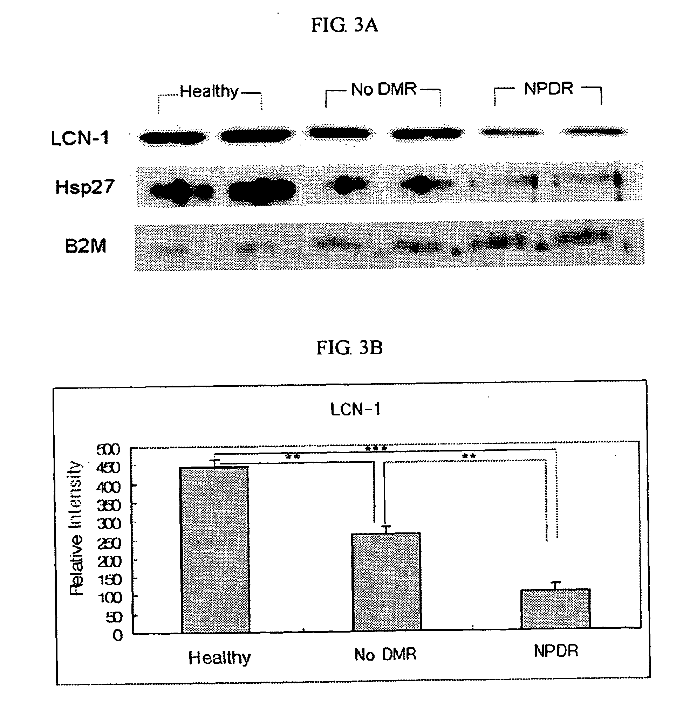

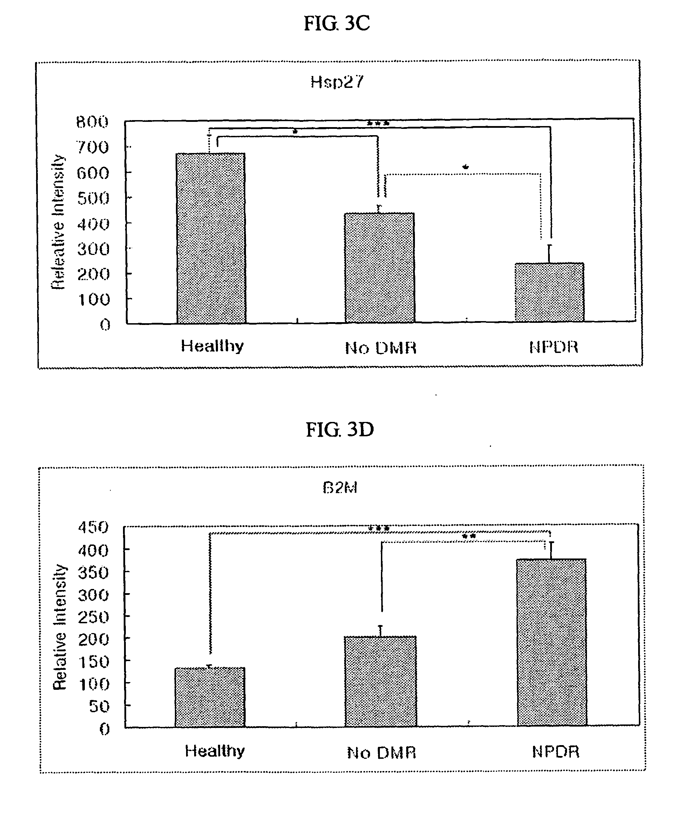

The results of western blot analysis of LCN-1, Hsp27 and B2M are shown in FIG 3A, and relative intensities of the proteins are shown in FIGs. 3B to 3D, respectively (In each bar, *, * and *** represents that significant differences between groups determined by unpaired student's t-test are p<0.05, p<0.01 and p<0.001, reapectively).

As shown in FIGs. 3A to 3D, the expression levels of LCN-1 and Hsp 27 were significantly down-regulated, and the expression level of B2M was up-regulated according to the progress of diabetic retinopathy. These results corresponds to the results of 2-DE Therefore, it is concluded that LCN-1, Hsp27 and B2M are useful as biomarkers for diagnosing diabetic retinopathy, and are useful treating or preventing diabetic retinopathy.

While the invention has been described with respect to the above specific embodiments, it should be recognized that various modifications and changes may be made to the invention by those skilled in the art which also fall within the scope of the invention as defined by the appended claims.
## Claims

1. An in vitro method of diagnosing diabetic retinopathy, which comprises measuring an expression level of a DJ-1 protein having a molecular weight of 24 to 26 kDa and an isoelectric point (PI) of 5.83 to 6.83, which comprises the peptide sequences of SEQ ID NOs: 1 and 2 obtained by trypsin digestion thereof, in the tears of a subject, and comparing the measured expression level to that measured in a healthy subject not suffering from diabetes.

2. The method of claim 1, which comprises
1) subjecting the tears obtained from a subject to a reaction with an antibody specific for the biomarker protein to obtain an antigen-antibody complex;
2) adding a secondary antibody conjugated with a marker, which will develop a color upon reaction with a substrate thereof, and a solution comprising the substrate to detect the antigen-antibody complex by color reaction; and
3) comparing the detected amount of the protein in the subject with that detected in a healthy subject not suffering from diabetes.

## Patentansprüche

1. In-vitro-Verfahren zum Diagnostizieren von diabetischer Retinopathie, welches umfasst, den Expressionsspiegel eines DJ-1-Proteins mit einem Molekulargewicht von 24 bis 26 kDa und einem isoelektrischen Punkt (pl) von 5,83 bis 6,83, das die Peptidsequenzen der SEQ ID NO: 1 und 2, erhalten durch Trypsinverdau desselben, umfasst, in den Tränen eines Probanden zu messen und den gemessenen Expressionsspiegel mit dem bei einem gesunden, nicht an Diabetes leidenden Probanden gemessenen zu vergleichen.

2. Verfahren nach Anspruch 1, welches umfasst
1) die von einem Probanden erhaltenen Tränen einer Reaktion mit einem Antikörper, der für das Biomarkerprotein spezifisch ist, zu unterziehen, um einen Antigen-Antikörper-Komplex zu erhalten;
2) einen mit einem Marker, der nach Reaktion mit einem Substrat desselben eine Farbe entwickelt, konjugierten sekundären Antikörper und eine das Substrat umfassende Lösung zuzugeben, um den Antigen-Antikörper-Komplex durch eine Farbreaktion nachzuweisen, und
3) die nachgewiesene Menge des Proteins bei dem Probanden mit der bei einem gesunden, nicht an Diabetes leidenden Probanden nachgewiesenen zu vergleichen.

## Revendications

1. Méthode in vitro pour diagnostiquer une rétinopathie diabétique, qui comprend la mesure d'un niveau d'expression d'une protéine DJ-1 présentant un poids moléculaire de 24 à 26 kDa et un point isoélectrique (PI) de 5,83 à 6,83, qui comprend les séquences peptidiques des SEQ ID NO : 1 et 2 obtenues par digestion à la trypsine de celle-ci, dans les larmes d'un sujet, et la comparaison du niveau d'expression mesuré avec celui mesuré chez un sujet en bonne santé ne souffrant pas de diabète.

2. Méthode selon la revendication 1, qui comprend
1) l'opération consistant à soumettre les larmes obtenues à partir d'un sujet à une réaction avec un anticorps spécifique de la protéine de biomarquage pour obtenir un complexe antigène-anticorps ;
2) l'addition d'un anticorps secondaire conjugué à un marqueur, qui va développer une couleur lors de sa réaction avec un substrat pour celui-ci, et une solution comprenant le substrat, pour détecter le complexe antigène-anticorps par réaction colorée ; et
3) la comparaison de la quantité détectée de la protéine chez le sujet avec celle détectée chez un sujet en bonne santé ne souffrant pas de diabète.


<110> Korea University Research and Business Foundation
<120> Bio-markers for diagnosing diabetic retinopathy
<130> EP63503HVpau
<140> not yet assigned
   <141> 2008-01-17
<150> KR 10-2008-0005162
   <151> 2008-01-17
<160> 50
<170> KopatentIn 1.71
<210> 1
   <211> 15
   <212> PRT
   <213> Homo sapiens
<400> 1
   Gly Ala Glu Glu Met Glu Thr Val Ile Pro Val Asp Val Met Arg
    1               5                  10                  15
<210> 2
   <211> 26
   <212> PRT
   <213> Homo sapiens
<400> 2
   Glu Gly Pro Tyr Asp Val Val Val Leu Pro Gly Gly Asn Leu Gly Ala
    1               5                  10                  15
   Gln Asn Leu Ser Glu Ser Ala Ala Val Lys
               20                  25
<210> 3
   <211> 22
   <212> PRT
   <213> Homo sapiens
<400> 3
   Ser Asn Phe Leu Asn Cys Tyr Val Ser Gly Phe His Pro Ser Asp Ile
    1               5                  10                  15
   Glu Val Asp Leu Leu Lys
               20
<210> 4
   <211> 23
   <212> PRT
   <213> Homo sapiens
<400> 4
   Asp Trp Ser Phe Tyr Leu Leu Tyr Tyr Thr Glu Phe Thr Pro Thr Glu
   1               5                   10                  15
   Lys Asp Glu Tyr Ala Cys Arg
               20
<210> 5
   <211> 13
   <212> PRT
   <213> Homo sapiens
<400> 5
   Met Ile Asn Ile Glu Ala Ser Gln Leu Ala Glu Val Arg
   1               5                   10
<210> 6
   <211> 16
   <212> PRT
   <213> Homo sapiens
<400> 6
   Ser Gly Leu Asn Thr Glu Ala Phe Tyr Val Met Thr Val Gly Ser Lys
   1               5                   10                  15
<210> 7
   <211> 18
   <212> PRT
   <213> Homo sapiens
<400> 7
   Ile Ile Pro Gly Gly Ile Tyr Asp Ala Asp Leu Asn Asp Glu Trp Val
   1               5                   10                  15
   Gln Arg
<210> 8
   <211> 11
   <212> PRT
   <213> Homo sapiens
<400> 8
   Gly Leu Ser Thr Glu Ser Ile Leu Ile Pro Arg
   1               5                   10
<210> 9
   <211> 16
   <212> PRT
   <213> Homo sapiens
<400> 9
   Phe Ala Ser Phe Ile Asp Lys Val Gln Phe Leu Glu Gln Gln Asn Lys
   1 5 10 15
<210> 10
   <211> 14
   <212> PRT
   <213> Homo sapiens
<400> 10
   Trp Asn Leu Leu Gln Gln Gln Thr Thr Thr Thr Ser Ser Lys
   1 5 10
<210> 11
   <211> 14
   <212> PRT
   <213> Homo sapiens
<400> 11
   Asn Leu Glu Pro Leu Phe Glu Thr Tyr Leu Ser Val Leu Arg
   1 5 10
<210> 12
   <211> 12
   <212> PRT
   <213> Homo sapiens
<400> 12
   Val Asp Ser Leu Asn Asp Glu Ile Asn Phe Leu Lys
   1 5 10
<210> 13
   <211> 12
   <212> PRT
   <213> Homo sapiens
<400> 13
   Asn Leu Asp Leu Asp Ser Ile Ile Ala Glu Val Arg
   1 5 10
<210> 14
   <211> 16
   <212> PRT
   <213> Homo sapiens
<400> 14
   Val Gln Gln Leu Gln Ile Ser Val Asp Gln His Gly Asp Asn Leu Lys
   1 5 10 15
<210> 15
   <211> 21
   <212> PRT
   <213> Homo sapiens
<400> 15
   Glu Leu Asp Ile Asn Thr Asp Gly Ala Val Asn Phe Gln Glu Phe Leu
   1 5 10 15
   Ile Leu Val Ile Lys
   20
<210> 16
   <211> 12
   <212> PRT
   <213> Homo sapiens
<400> 16
   Gln Asn Gln Glu Tyr Gln Val Leu Leu Asp Val Arg
   1 5 10
<210> 17
   <211> 15
   <212> PRT
   <213> Homo sapiens
<400> 17
   Leu Asn Val Glu Val Asp Ala Ala Pro Thr Val Asp Leu Asn Arg
   1 5 10 15
<210> 18
   <211> 10
   <212> PRT
   <213> Homo sapiens
<400> 18
   Leu Phe Asp Gln Ala Phe Gly Leu Pro Arg
   1 5 10
<210> 19
   <211> 16
   <212> PRT
   <213> Homo sapiens
<400> 19
   Val Ser Leu Asp Val Asn His Phe Ala Pro Asp Glu Leu Thr Val Lys
   1 5 10 15
<210> 20
   <211> 17
   <212> PRT
   <213> Homo sapiens
<400> 20
   Leu Ala Thr Gln Ser Asn Glu Ile Thr Ile Pro Val Thr Phe Glu Ser
   1               5                   10                  15
   Arg
<210> 21
   <211> 18
   <212> PRT
   <213> Homo sapiens
<400> 21
   Leu Gln Ala Glu Val Leu Glu Cys Val Ser Leu Val Glu Leu Thr Ser
   1               5                   10                  15
   Leu Lys
<210> 22
   <211> 11
   <212> PRT
   <213> Homo sapiens
<400> 22
   Ala Leu Ile Pro Asp Val Asp Leu Asp Ser Asp
   1               5                   10
<210> 23
   <211> 10
   <212> PRT
   <213> Homo sapiens
<400> 23
   Leu Leu Val Val Tyr Pro Trp Thr Gln Arg
   1               5                   10
<210> 24
   <211> 12
   <212> PRT
   <213> Homo sapiens
<400> 24
   Glu Phe Thr Pro Pro Val Gln Ala Ala Tyr Gln Lys
   1               5                   10
<210> 25
   <211> 19
   <212> PRT
   <213> Homo sapiens
<400> 25
   Phe Phe Glu Ser Phe Gly Asp Leu Ser Thr Pro Asp Ala Val Met Gly
   1 5 10 15
   Asn Pro Lys
<210> 26
   <211> 12
   <212> PRT
   <213> Homo sapiens
<400> 26
   Gly Ile Ser Leu Ala Asn Trp Met Cys Leu Ala Lys
   1 5 10
<210> 27
   <211> 12
   <212> PRT
   <213> Homo sapiens
<400> 27
   Ser Thr Asp Tyr Gly Ile Phe Gln Ile Asn Ser Arg
   1 5 10
<210> 28
   <211> 13
   <212> PRT
   <213> Homo sapiens
<400> 28
   Gln Leu Ser Phe Glu Glu Phe Ile Met Leu Met Ala Arg
   1 5 10
<210> 29
   <211> 15
   <212> PRT
   <213> Homo sapiens
<400> 29
   Asn Ile Glu Thr Ile Ile Asn Thr Phe His Gln Tyr Ser Val Lys
   1 5 10 15
<210> 30
   <211> 28
   <212> PRT
   <213> Homo sapiens
<400> 30
   Val Ile Glu His Ile Met Glu Asp Leu Asp Thr Asn Ala Asp Lys Gln
   1 5 10 15
   Leu Ser Phe Glu Glu Phe Ile Met Leu Met Ala Arg
   20 25
<210> 31
   <211> 13
   <212> PRT
   <213> Homo sapiens
<400> 31
   Gln Leu Ser Phe Glu Glu Phe Ile Met Leu Met Ala Arg
   1 5 10
<210> 32
   <211> 11
   <212> PRT
   <213> Homo sapiens
<400> 32
   Ala Leu Asp Val Met Val Ser Thr Phe His Lys
   1 5 10
<210> 33
   <211> 188
   <212> PRT
   <213> Homo sapiens
<400> 33
   Met Ala Ser Lys Arg Ala Leu Val Ile Leu Ala Lys Gly Ala Glu Glu
   1               5                   10                  15
   Met Glu Thr Val Ile Pro Val Asp Val Met Arg Arg Ala Gly Ile Lys
               20                  25                  30
   Val Thr Val Ala Gly Leu Ala Gly Lys Asp Pro Val Gln Cys Ser Arg
           35                  40                  45
   Asp Val Val Ile Cys Pro Asp Ala Ser Leu Glu Asp Ala Lys Lys Glu
       50                  55                  60
   Gly Pro Tyr Asp Val Val Val Leu Pro Gly Gly Asn Leu Gly Ala Gln
   65                  70                  75                  80
   Asn Leu Ser Glu Ser Ala Ala Val Lys Glu Ile Leu Lys Glu Gln Glu
                   85                  90                  95
   Asn Arg Lys Gly Leu Ile Ala Ala Ile Cys Ala Gly Pro Thr Ala Leu
               100                 105                 110
   Leu Ala His Glu Ile Gly Phe Gly Ser Lys Val Thr Thr His Pro Leu
           115                 120                 125
   Ala Lys Asp Lys Met Met Asn Gly Gly His Tyr Thr Tyr Ser Glu Asn
       130                 135                 140
   Arg Val Glu Lys Asp Leu Ile Leu Thr Ser Arg Gly Pro Gly Thr Ser
   145                 150                 155                 160
   Phe Glu Phe Ala Leu Ala Ile Val Glu Ala Leu Asn Gly Lys Glu Val
                   165                 170                 175
   Ala Ala Gln Val Lys Ala Pro Leu Val Leu Lys Asp
               180                 185
<210> 34
   <211> 110
   <212> PRT
   <213> Homo sapiens
<400> 34
   Leu Ala Leu Leu Ser Leu Ser Gly Leu Glu Ala Ile Gln Arg Thr Pro
   1               5                   10                  15
   Lys Ile Gln Val Tyr Ser Arg His Pro Ala Glu Asn Gly Lys Ser Asn
               20                  25                  30
   Phe Leu Asn Cys Tyr Val Ser Gly Phe His Pro Ser Asp Ile Glu Val
           35                  40                  45
   Asp Leu Leu Lys Asn Gly Glu Arg Ile Glu Lys Val Glu His Ser Asp
       50                  55                  60
   Leu Ser Phe Ser Lys Asp Trp Ser Phe Tyr Leu Leu Tyr Tyr Thr Glu
   65                  70                  75                  80
   Phe Thr Pro Thr Glu Lys Asp Glu Tyr Ala Cys Arg Val Asn His Val
                   85                  90                  95
   Thr Leu Ser Gln Pro Lys Ile Val Lys Trp Asp Arg Asp Met
               100                 105                 110
<210> 35
   <211> 500
   <212> PRT
   <213> Homo sapiens
<400> 35
   Phe Asn Cys Leu Gly Met Gly Asn Arg Asp Phe Ile Glu Gly Ala Ser
   1               5                   10                  15
   Gly Ala Thr Trp Val Asp Leu Val Leu Glu Gly Asp Ser Cys Leu Thr
               20                  25                  30
   Ile Met Ala Asn Asp Lys Pro Thr Leu Asp Val Arg Met Ile Asn Ile
           35                  40                  45
   Glu Ala Ser Gln Leu Ala Glu Val Arg Ser Tyr Cys Tyr His Ala Ser
       50                  55                  60
   Val Thr Asp Ile Ser Thr Val Ala Arg Cys Pro Thr Thr Gly Glu Ala
   65                  70                  75                  80
   His Asn Glu Lys Arg Ala Asp Ser Ser Tyr Val Cys Lys Gln Gly Phe
                   85                  90                  95
   Thr Asp Arg Gly Trp Gly Asn Gly Cys Gly Leu Phe Gly Lys Gly Ser
               100                 105                 110
   Ile Asp Thr Cys Ala Lys Phe Ser Cys Thr Ser Lys Ala Ile Gly Arg
           115                 120                 125
   Thr Ile Gln Pro Glu Asn Ile Lys Tyr Glu Val Gly Ile Phe Val His
       130                 135                 140
   Gly Thr Thr Thr Ser Glu Asn His Gly Asn Tyr Ser Ala Gln Val Gly
   145                 150                 155                 160
   Ala Ser Gln Ala Ala Lys Phe Thr Val Thr Pro Asn Ala Pro Ser Ile
                   165                 170                 175
   Thr Leu Lys Leu Gly Asp Tyr Gly Glu Val Thr Leu Asp Cys Glu Pro
               180                 185                 190
   Arg Ser Gly Leu Asn Thr Glu Ala Phe Tyr Val Met Thr Val Gly Ser
   195 200 205
   Lys Ser Phe Leu Val His Arg Glu Trp Phe His Asp Leu Ala Leu Pro
   210 215 220
   Trp Thr Ser Pro Ser Ser Thr Ala Trp Arg Asn Arg Glu Leu Leu Met
   225 230 235 240
   Glu Phe Glu Glu Ala His Ala Thr Lys Gln Ser Val Val Ala Leu Gly
   245 250 255
   Ser Gln Glu Gly Gly Leu His Gln Ala Leu Ala Gly Ala Ile Val Val
   260 265 270
   Glu Tyr Ser Ser Ser Val Lys Leu Thr Ser Gly His Leu Lys Cys Arg
   275 280 285
   Leu Lys Met Asp Lys Leu Ala Leu Lys Gly Thr Thr Tyr Gly Met Cys
   290 295 300
   Thr Glu Lys Phe Ser Phe Ala Lys Asn Pro Ala Asp Thr Gly His Gly
   305 310 315 320
   Thr Val Val Ile Glu Leu Ser Tyr Ser Gly Ser Asp Gly Pro Cys Lys
   325 330 335
   Ile Pro Ile Val Ser Val Ala Ser Leu Asn Asp Met Thr Pro Val Gly
   340 345 350
   Arg Leu Val Thr Val Asn Pro Phe Val Ala Thr Ser Ser Ala Asn Ser
   355 360 365
   Lys Val Leu Val Glu Met Glu Pro Pro Phe Gly Asp Ser Tyr Ile Val
   370 375 380
   Val Gly Arg Gly Asp Lys Gln Ile Asn His His Trp His Lys Ala Gly
   385 390 395 400
   Ser Thr Leu Gly Lys Ala Phe Ser Thr Thr Leu Lys Gly Ala Gln Arg
   405 410 415
   Leu Ala Ala Leu Gly Asp Thr Ala Trp Asp Phe Gly Ser Ile Gly Gly
   420 425 430
   Val Phe Asn Ser Ile Gly Lys Ala Val His Gln Val Phe Gly Gly Ala
   435 440 445
   Phe Arg Thr Leu Phe Gly Gly Met Ser Trp Ile Thr Gln Gly Leu Met
   450 455 460
   Gly Ala Leu Leu Leu Trp Met Gly Val Asn Ala Arg Asp Arg Ser Ile
   465 470 475 480
   Ala Leu Ala Phe Leu Ala Thr Gly Gly Val Leu Val Phe Leu Ala Thr
   485 490 495
   Asn Val His Ala
   500
<210> 36
   <211> 113
   <212> PRT
   <213> Homo sapiens
<400> 36
<210> 37
   <211> 162
   <212> PRT
   <213> Homo sapiens
<400> 37
   Met Lys Pro Leu Leu Leu Ala Ile Ser Leu Ser Leu Ile Ala Ala Leu
   1               5                   10                  15
   Gln Ala His His Leu Leu Ala Ser Asp Glu Glu Ile Gln Asp Val Ser
               20                  25                  30
   Gly Thr Trp Tyr Leu Lys Ala Met Thr Val Asp Arg Glu Leu Pro Glu
           35                  40                  45
   Met Asn Leu Glu Ser Val Thr Pro Met Thr Leu Thr Ile Leu Glu Gly
       50                  55                  60
   Gly Asn Leu Glu Ala Lys Ala Thr Met Leu Ile Ser Gly Gln Cys Gln
   65                  70                  75                  80
   Glu Val Lys Val Ile Leu Glu Lys Thr Asp Glu Pro Gly Lys Tyr Thr
                   85                  90                  95
   Ala Asn Arg Gly Lys His Val Ala Tyr Ile Ile Arg Ser His Met Lys
               100                 105                 110
   Asp His Tyr Ile Phe Tyr Cys Glu Gly Arg Asp Pro Glu Asn Asn Leu
           115                 120                 125
   Glu Ala Leu Glu Asp Phe Glu Lys Ala Ala Gly Ala Arg Gly Leu Ser
       130                 135                 140
   Thr Glu Ser Ile Leu Ile Pro Arg Gln Ser Glu Thr Cys Ser Pro Gly
   145                 150                 155                 160
   Ser Asp
<210> 38
   <211> 176
   <212> PRT
   <213> Homo sapiens
<400> 38
<210> 39
   <211> 408
   <212> PRT
   <213> Homo sapiens
<400> 39
   Ser Leu Asn Gln Ser Leu Leu Thr Pro Leu His Val Glu Ile Asp Pro
   1               5                   10                  15
   Glu Ile Gln Lys Val Arg Thr Glu Glu Arg Glu Gln Ile Lys Leu Leu
               20                  25                  30
   Asn Asn Lys Phe Ala Ser Phe Ile Asp Lys Val Gln Phe Leu Glu Gln
           35                  40                  45
   Gln Asn Lys Val Leu Glu Thr Lys Trp Asn Leu Leu Gln Gln Gln Thr
       50                  55                  60
   Thr Thr Thr Ser Ser Lys Asn Leu Glu Pro Leu Phe Glu Thr Tyr Leu
   65                  70                  75                  80
   Ser Val Leu Arg Lys Gln Leu Asp Thr Leu Gly Asn Asp Lys Gly Arg
                   85                  90                  95
   Leu Gln Ser Glu Leu Lys Thr Met Gln Asp Ser Val Glu Asp Phe Lys
               100                 105                 110
   Thr Lys Tyr Glu Glu Glu Ile Asn Lys Arg Thr Ala Ala Glu Asn Asp
           115                 120                 125
   Phe Val Val Leu Lys Lys Asp Val Asp Ala Ala Tyr Leu Asn Lys Val
       130                 135                 140
   Glu Leu Glu Ala Lys Val Asp Ser Leu Asn Asp Glu Ile Asn Phe Leu
   145 150 155 160
   Lys Val Leu Tyr Asp Ala Glu Leu Ser Gln Met Gln Thr His Val Ser
   165 170 175
   Asp Thr Ser Val Val Leu Ser Met Asp Asn Asn Arg Asn Leu Asp Leu
   180 185 190
   Asp Ser Ile Ile Ala Glu Val Arg Ala Gln Tyr Glu Glu Ile Ala Gln
   195 200 205
   Arg Ser Lys Ala Glu Ala Glu Ala Leu Tyr Gln Thr Lys Val Gln Gln
   210 215 220
   Leu Gln Ile Ser Val Asp Gln His Gly Asp Asn Leu Lys Asn Thr Lys
   225 230 235 240
   Ser Glu Ile Ala Glu Leu Asn Arg Met Ile Gln Arg Leu Arg Ala Glu
   245 250 255
   Ile Glu Asn Ile Lys Lys Gln Cys Gln Thr Leu Gln Val Ser Val Ala
   260 265 270
   Asp Ala Glu Gln Arg Gly Glu Asn Ala Leu Lys Asp Ala His Ser Lys
   275 280 285
   Arg Val Glu Leu Glu Ala Ala Leu Gln Gln Ala Lys Glu Glu Leu Ala
   290 295 300
   Arg Met Leu Arg Glu Tyr Gln Glu Leu Met Ser Val Lys Leu Ala Leu
   305 310 315 320
   Asp Ile Glu Ile Ala Thr Tyr Arg Lys Leu Leu Glu Gly Glu Glu Tyr
   325 330 335
   Arg Met Ser Gly Glu Cys Gln Ser Ala Val Ser Ile Ser Val Val Ser
   340 345 350
   Gly Ser Thr Ser Thr Gly Gly Ile Ser Gly Gly Leu Gly Ser Gly Ser
   355 360 365
   Gly Phe Gly Leu Ser Ser Gly Phe Gly Ser Gly Ser Gly Ser Gly Phe
   370 375 380
   Gly Phe Gly Gly Ser Val Ser Gly Ser Ser Ser Ser Lys Ile Ile Ser
   385 390 395 400
   Thr Thr Thr Leu Asn Lys Arg Arg
   405
<210> 40
   <211> 93
   <212> PRT
   <213> Homo sapiens
<400> 40
   Met Leu Thr Glu Leu Glu Lys Ala Leu Asn Ser Ile Ile Asp Val Tyr
   1 5 10 15
   His Lys Tyr Ser Leu Ile Lys Gly Asn Phe His Ala Val Tyr Arg Asp
   20 25 30
   Asp Leu Lys Lys Leu Leu Glu Thr Glu Cys Pro Gln Tyr Ile Arg Lys
   35 40 45
   Lys Gly Ala Asp Val Trp Phe Lys Glu Leu Asp Ile Asn Thr Asp Gly
   50 55 60
   Ala Val Asn Phe Gln Glu Phe Leu Ile Leu Val Ile Lys Met Gly Val
   65 70 75 80
   Ala Ala His Lys Lys Ser His Glu Glu Ser His Lys Glu
   85 90
<210> 41
   <211> 176
   <212> PRT
   <213> Homo sapiens
<400> 41
   Met Lys Pro Leu Leu Leu Ala Val Ser Leu Gly Leu Ile Ala Ala Leu
   1 5 10 15
   Gln Ala His His Leu Leu Ala Ser Asp Glu Glu Ile Gln Asp Val Ser
   20 25 30
   Gly Thr Trp Tyr Leu Lys Ala Met Thr Val Asp Arg Glu Phe Pro Glu
   35 40 45
   Met Asn Leu Glu Ser Val Thr Pro Met Thr Leu Thr Thr Leu Glu Gly
   50 55 60
   Gly Asn Leu Glu Ala Lys Val Thr Met Leu Ile Ser Gly Arg Cys Gln
   65 70 75 80
   Glu Val Lys Ala Val Leu Glu Lys Thr Asp Glu Pro Gly Lys Tyr Thr
   85 90 95
   Ala Asp Gly Gly Lys His Val Ala Tyr Ile Ile Arg Ser His Val Lys
   100 105 110
   Asp His Tyr Ile Phe Tyr Cys Glu Gly Glu Leu His Gly Lys Pro Val
   115 120 125
   Arg Gly Val Lys Leu Val Gly Arg Asp Pro Lys Asn Asn Leu Glu Ala
   130 135 140
   Leu Glu Asp Phe Glu Lys Ala Ala Gly Ala Arg Gly Leu Ser Thr Glu
   145 150 155 160
   Ser Ile Leu Ile Pro Arg Gln Ser Glu Thr Cys Ser Pro Gly Ser Asp
   165 170 175
<210> 42
   <211> 416
   <212> PRT
   <213> Homo sapiens
<400> 42
<210> 43
   <211> 199
   <212> PRT
   <213> Homo sapiens
<400> 43
   Met Thr Glu Arg Arg Val Pro Phe Ser Leu Leu Arg Gly Pro Ser Trp
   1               5                   10                  15
   Asp Pro Phe Arg Asp Trp Tyr Pro His Ser Arg Leu Phe Asp Gln Ala
               20                  25                  30
   Phe Gly Leu Pro Arg Leu Pro Glu Glu Trp Ser Gln Trp Leu Gly Gly
           35                  40                  45
   Ser Ser Trp Pro Gly Tyr Val Arg Pro Leu Pro Pro Ala Ala Ile Glu
       50                  55                  60
   Ser Pro Ala Val Ala Ala Pro Ala Tyr Ser Arg Ala Leu Ser Arg Gln
   65                  70                  75                  80
   Leu Ser Ser Gly Val Ser Glu Ile Arg His Thr Ala Asp Arg Trp Arg
                   85                  90                  95
   Val Ser Leu Asp Val Asn His Phe Ala Pro Asp Glu Leu Thr Val Lys
               100                 105                 110
   Thr Lys Asp Gly Val Val Glu Ile Thr Gly Lys His Glu Glu Arg Gln
           115                 120                 125
   Asp Glu His Gly Tyr Ile Ser Arg Cys Phe Thr Arg Lys Tyr Thr Leu
       130                 135                 140
   Pro Pro Gly Val Asp Pro Thr Gln Val Ser Ser Ser Leu Ser Pro Glu
   145                 150                 155                 160
   Gly Thr Leu Thr Val Glu Ala Pro Met Pro Lys Leu Ala Thr Gln Ser
                   165                 170                 175
   Asn Glu Ile Thr Ile Pro Val Thr Phe Glu Ser Arg Ala Gln Leu Gly
               180                 185                 190
   Gly Arg Ser Cys Lys Ile Arg
           195
<210> 44
   <211> 180
   <212> PRT
   <213> Homo sapiens
<400> 44
<210> 45
   <211> 125
   <212> PRT
   <213> Homo sapiens
<400> 45
   Met Ala Val Ala Asp Leu Ala Leu Ile Pro Asp Val Asp Ile Asp Ser
   1               5                   10                  15
   Asp Gly Val Phe Lys Tyr Val Leu Ile Arg Val His Ser Ala Pro Arg
               20                  25                  30
   Ser Gly Ala Pro Ala Ala Glu Ser Lys Glu Ile Val Arg Gly Tyr Lys
           35                  40                  45
   Trp Ala Glu Tyr His Ala Asp Ile Tyr Asp Lys Val Ser Gly Asp Met
       50                  55                  60
   Gln Lys Gln Gly Cys Asp Cys Glu Cys Leu Gly Gly Gly Arg Ile Ser
   65                  70                  75                  80
   His Gln Ser Gln Asp Lys Lys Ile His Val Tyr Gly Tyr Ser Met Ala
                   85                  90                  95
   Tyr Gly Pro Ala Gln His Ala Ile Ser Thr Glu Lys Ile Lys Ala Lys
               100                 105                 110
   Tyr Pro Asp Tyr Glu Val Thr Trp Ala Asn Asp Gly Tyr
           115                 120                 125
<210> 46
   <211> 147
   <212> PRT
   <213> Homo sapiens
<400> 46
<210> 47
   <211> 148
   <212> PRT
   <213> Homo sapiens
<400> 47
<210> 48
   <211> 114
   <212> PRT
   <213> Homo sapiens
<400> 48
   Met Thr Cys Lys Met Ser Gln Leu Glu Arg Asn Ile Glu Thr Ile Ile
   1               5                   10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 49
   <211> 114
   <212> PRT
   <213> Homo sapiens
<400> 49
   Met Thr Cys Lys Met Ser Gln Leu Glu Arg Asn Ile Glu Thr Ile Ile
   1               5                   10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 50
   <211> 101
   <212> PRT
   <213> Homo sapiens
<400> 50
   Met Ala Cys Pro Leu Glu Lys Ala Leu Asp Val Met Val Ser Thr Phe
   1               5                   10                  15
   His Lys Tyr Ser Gly Lys Glu Gly Asp Lys Phe Lys Leu Asn Lys Ser
               20                  25                  30
   Glu Leu Lys Glu Leu Leu Thr Arg Glu Leu Pro Ser Phe Leu Gly Lys
           35                  40                  45
   Arg Thr Asp Glu Ala Ala Phe Gln Lys Leu Met Ser Asn Leu Asp Ser
       50                  55                  60
   Asn Arg Asp Asn Glu Val Asp Phe Gln Glu Tyr Cys Val Phe Leu Ser
   65                  70                  75                  80
   Cys Ile Ala Met Met Cys Asn Glu Phe Phe Glu Gly Phe Pro Asp Lys
                   85                  90                  95
   Gln Pro Arg Lys Lys
               100